# EUROPÄISCHE PATENTSCHRIFT

(11) **EP 2 781 206 B1**
(45) Veröffentlichungstag und Bekanntmachung des Hinweises auf die Patenterteilung: **23.11.2016**
(21) Anmeldenummer: 14155453.5
(22) Anmeldetag: 17.02.2014
(51) Int. Cl.: A61F 2/38, A61F 2/30

(54) **Zweiteiliger Kniespacer mit Aussparungen**
Two-part knee spacer with recesses
Espaceur de genou en deux parties avec évidements

(30) Priorität: 22.03.2013 DE 102013205156
(43) Veröffentlichungstag der Anmeldung: 24.09.2014
(73) Patentinhaber: Heraeus Medical GmbH, 61273 Wehrheim (DE)
(72) Erfinder: Vogt, Sebastian, 99092 Erfurt (DE)
(74) Vertreter: Schultheiss & Sterzel Patentanwälte PartG mbB

(56) Entgegenhaltungen:
- WO-A1-01/76512
- US-A1- 2004 153 162
- US-A1- 2010 102 484
- US-A1- 2013 073 049

## Beschreibung

Die Erfindung betrifft einen Kniespacer zum zeitweisen Ersetzen eines künstlichen Kniegelenks, wobei der Kniespacer eine Tibiakomponente und eine Femurkomponente als separate und, im beim Patienten eingesetzten Zustand, gegeneinander bewegliche Komponenten aufweist. Die Erfindung betrifft auch ein Verfahren zur Herstellung eines Kniespacers und die Verwendung eines solchen Kniespacers.

Gegenstand der Erfindung ist also ein zweiteiliger Kniespacer beziehungsweise ein zweiteiliges Kniespacersystem, das als temporärer Platzhalter im Rahmen von zweizeitigen septischen Revisionen von Kniegelenkendoprothesen verwendet wird.

Gelenkendoprothesen haben gegenwärtig eine Standzeit von mehreren Jahren, zum Beispiel bei zementierten Hüftgelenkprothesen im Durchschnitt größer als zehn bis fünfzehn Jahre. Es gibt jedoch unerwünschte Lockerungen der Gelenkendoprothesen, die vor dem Erreichen der üblichen Standzeiten auftreten. Es wird dabei die septische und die aseptische Lockerung unterschieden. Bei der aseptischen Lockerung lassen sich bisher keine mikrobiellen Keime nachweisen. Die Ursachen der aseptischen Lockerungen können vielfältig sein. Häufig sind aseptische Lockerungen auf Abrieb an den Gleitflächen der Gelenkendoprothesen zurückzuführen.

Bei der septischen Lockerung wird der Lockerungsprozess durch mikrobielle Keime hervorgerufen. Man unterscheidet dabei in Abhängigkeit vom zeitlichen Auftreten frühe und späte Infektionen. Die septische Lockerung ist eine sehr ernste Erkrankung für den Patienten, die zusätzlich bei der Behandlung mit sehr hohen Kosten verbunden ist. Sowohl bei der aseptischen als auch der septischen Lockerung wird üblicherweise eine Revision vorgenommen. Man unterscheidet einzeitige und zweizeitige Revisionen. Bei septischen Lockerungen werden sehr häufig zweizeitige Revisionen vorgenommen.

Bei der zweizeitigen Revision wird in einer ersten Operation (OP) die infizierte Gelenkendoprothese entfernt, es erfolgt ein Debridement (ein Entfernen des infizierten Gewebes) und anschließend wird ein temporärer Platzhalter, ein so genannter Spacer, eingesetzt. Dieser Spacer füllt für einige Wochen den Raum der zuvor revidierten Endoprothese aus, bis die vorliegende Infektion abgeklungen ist. Diese Platzhalterfunktion ist sehr wichtig, um eine Atrophie der Muskulatur in dieser Zeit wirksam zu verhindern und um eine Stabilisierung der Resektionssituation zu erreichen.

Man unterscheidet nichtartikulierende und artikulierende Spacer. Artikulierende Spacer bilden die Gelenkfunktion nach und erlauben eine gewisse Beweglichkeit der betroffenen Gliedmaßen. Es ist dadurch möglich, die Patienten frühzeitig zu mobilisieren. Artikulierende Spacer werden daher derzeit besonders gerne eingesetzt. Der Spacer wird in einer zweiten OP entfernt, es wird nochmals debridiert und dann wird eine zementierte oder auch zementfreie Revisions-Gelenkendoprothese implantiert.

Die Verwendung von Spacern geht ursprünglich auf Hovelius und Josefsson zurück (Hovelius L, Josefsson G (1979), "An alternative method for exchange operation of infected arthroplasty", Acta Orthop. Scand. 50: 93-96). Weitere frühe Arbeiten zu Spacern stammen von Younger (Younger AS, Duncan CP, Masri BA, McGraw RW (1997), "The outcome of two-stage arthroplasty using a custom-made interval spacer to treat the infected hip", J. Arthroplasty 12: 615-623), Jones (Jones WA, Wroblewski BM (1989), "Salvage of failed total knee arthroplasty: the 'beefburger' procedure", J. Bone Joint Surg. Br. 71: 856-857.) und Cohen (Cohen JC, Hozack WJ, Cuckler JM, Booth RE Jr (1988), "Two-stage reimplantation of septic total knee arthroplasty, Report of three cases using an antibiotic-PMMA spacer block", J. Arthroplasty 3: 369-377). Von McPherson stammt die Konzeption, dass Spacer ausschließlich aus Knochenzement hergestellt werden können (McPherson EJ, Lewonowski K, Dorr LD (1995), "Techniques in arthroplasty. Use of an articulated PMMA spacer in the infected total knee arthroplasty", J. Arthroplasty 10: 87-89).

Aus der US 2004/0153162 A1 ist eine zweiteilige provisorische Knieprothese bekannt, die auf einem nur teilweise vorbereiteten Knochen befestigt werden kann. Die Femurkomponente weist dazu Aussparungen auf.

Es gibt auf dem Markt mit Antibiotika ausgerüstete Spacer zum temporären Ersatz von Knie-, Hüft- und Schultergelenkendoprothesen. Kniespacer sind im Allgemeinen zweiteilig und bestehen aus einer Tibiakomponente und einer Femurkomponente. Ein dafür typischer Spacer beziehungsweise ein dafür typisches Spacersystem ist in der EP 1 274 374 A1 offenbart. Im Fall der Kniespacer müssen zusätzlich sowohl die Tibiakomponente als auch die Femurkomponente mit Polymethylmethacrylat-Knochenzement an der proximalen Tibia und am distalen Femur verankert werden. Dabei haftet der Polymethylmethacrylat-Knochenzement an der Oberfläche der Spacerkomponenten. Die Oberfläche der Spacerkomponenten, die im Allgemeinen aus ausgehärtetem Antibiotika-dotiertem Polymethylmethacrylat-Knochenzement bestehen, wird durch das Methylmethacrylat des Polymethylmethacrylat-Knochenzements mehr oder weniger angelöst. Dadurch kommt es zum Verbund mit dem aushärtenden Polymethylmethacrylat-Knochenzement. Darauf beruht im Wesentlichen die Haftung des Polymethylmethacrylat-Knochenzements auf der Oberfläche der Spacerkomponenten. Problematisch ist jedoch, dass während der Implantationsdauer, die je nach Behandlungskonzept zwischen zwei Wochen bis maximal sechs Monate andauern kann, auf Grund der Gehbewegung der Patienten die Spacerkomponenten sich durch Torsionskräfte vom zur Verankerung verwendeten Polymethylmethacrylat-Knochenzement ablösen können.

Die Aufgabe der Erfindung besteht also darin, die Nachteile des Stands der Technik zu überwinden. Insbesondere soll ein stabiler artikulierender Kniespacer bereitgestellt werden, mit dem robuste Verbindungen zum Femur und zur Tibia herstellbar sind. Der Spacer soll besonders gut beweglich sein und auch bei häufiger Bewegung stabil bleiben. Gleichzeitig soll der Spacer kostengünstig in der Fertigung sein.

Die Aufgaben der Erfindung werden gelöst durch einen Kniespacer zum zeitweisen Ersetzen eines künstlichen Kniegelenks, wobei der Kniespacer eine Tibiakomponente und eine Femurkomponente als separate und, im beim Patienten eingesetzten Zustand, gegeneinander bewegliche Komponenten aufweist und die Tibiakomponente und die Femurkomponente jeweils zumindest eine Lauffläche aufweisen, über die die Tibiakomponente und die Femurkomponente für den beim Patienten eingesetzten Zustand beweglich aneinander anlegbar sind, und die Tibiakomponente eine Verankerungsfläche aufweist, die der Laufflächenseite gegenüber liegend angeordnet ist und die zur Verbindung der Tibiakomponente mit der Tibia mittels eines Knochenzements vorgesehen ist und die Femurkomponente eine Verankerungsfläche aufweist, die der Laufflächenseite gegenüber liegend angeordnet ist und die zur Verbindung der Femurkomponente mit dem Femur vorgesehen ist, wobei die Tibiakomponente und die Femurkomponente jeweils zumindest zwei Aussparungen aufweist, die sich von der Verankerungsfläche in Richtung der Laufflächenseite bis in die Laufflächenseite erstrecken, wobei die Öffnungen der Aussparungen der Komponenten in der Laufflächenseite derart angeordnet sind, dass sie beim Artikulieren des Kniespacers nicht über die Lauffläche der jeweils anderen Komponente gleiten.

Mit dem beim Patienten eingesetzten Zustand ist die Anordnung gemeint, bei der die Komponenten des Kniespacers im Knie des Patienten einzuzementieren sind beziehungsweise einzementiert werden sollen, um ein funktionstüchtiges, das heißt bewegliches temporäres Implantat zu bilden.

Durch die Länge der Aussparungen bis zur Laufflächenseite können besonders langgestreckte und dadurch nach dem Aushärten stabile Ausläufer des Knochenzements gebildet werden, die die Komponenten halten.

Durch die Vermeidung des Übergleitens der laufflächenseitigen Öffnungen der Aussparungen bei Artikulieren des Kniespacers, das heißt bei einer vorgesehenen Drehung der beim Patienten eingesetzten und aneinander anliegenden Komponenten gegeneinander, kann vermieden werden, dass sich Teile des Knochenzements, mit dem die Komponenten befestigt sind und die sich durch die Aussparungen bis in die laufflächenseitigen Öffnungen erstrecken, durch das Übergleiten der Komponenten abreiben. Dadurch kann vermieden werden, dass Teile des Abriebs zu Beeinträchtigungen im Kniebereich und/oder zu einer Verschlechterung der Artikulation des Kniespacers führen.

Bei erfindungsgemäßen Kniespacern kann vorgesehen sein, dass die Aussparungen randseitig in der Tibiakomponente und der Femurkomponente angeordnet sind.

Durch die randseitige Anordnung der Aussparrungen entstehen über die langen Hebel bis zu den Aussparungen besonders robuste Verbindungen der Komponenten zu dem Knochenzement, mit dem die Komponenten am Knochen befestigt werden. Zudem können die beim Gehen auftretenden Drehmomente und Kräfte durch die langen Hebel besonders gut aufgenommen werden.

Erfindungsgemäß bevorzugt kann auch vorgesehen sein, dass zumindest zwei der Aussparungen derart zueinander gegenüber liegend angeordnet sind, dass, im beim Patienten eingesetzten Zustand der Komponenten, durch den Knochenzement in den Aussparungen geformte Ausläufer die Komponenten derart in die Zange nehmen, dass Bereiche der Komponenten, bevorzugt zentrale Bereiche der Komponenten zwischen den Ausläufern angeordnet sind.

Durch diese Anordnung kann eine weitere Stabilisierung der Verbindung der Komponenten zum Knochenzement und erreicht werden. In die Zange nehmen bedeutet, dass über die Innenwände zweier Aussparungen entgegengesetzte Kräfte wirken können. Die Kräfte wirken dabei bevorzugt durchgehend im Inneren der Komponenten. Mit diesen Kräften werden eine besonders stabile Befestigung der Komponenten und auch eine variable Kraftaufnahme beim Gehen erreicht. Die Ausläufer werden von dem Knochenzement gebildet, der die Komponenten am Knochen befestigt. Die Ausläufer des Knochenzements bilden dadurch einen U-förmigen Eingriff in jeweils zwei Aussparungen.

Ferner kann vorgesehen sein, dass sich die Aussparungen nicht in die Lauffläche erstrecken.

Hierdurch wird ein Abrieb von Teilen des Knochenzements, die sich bis zur Lauffläche erstrecken könnten, beim Artikulieren des Kniespacers vermieden.

Mit einer Weiterbildung der Erfindung wird vorgeschlagen, dass in den Aussparungen Vorsprünge zur Verankerung des Knochenzements angeordnet sind.

Mit den Vorsprüngen, die von dem Knochenzement zur Befestigung der Komponenten an der Tibia beziehungsweise dem Femur umflossen werden können, wir eine besonders stabile Verankerung der Komponenten am Knochen erreicht.

Mit einer besonders bevorzugten Weiterentwicklung der Erfindung kann vorgesehen sein, dass die Tibiakomponente und/oder die Femurkomponente asymmetrisch zu einer Ebene im eingesetzten Zustand ist oder sind, die parallel zur Sagittalebene des Patienten ist, vorzugsweise durch die Anordnung und/oder Form einer Aussparung zur Aufnahme des Kreuzbands des Patienten asymmetrisch ist oder sind und/oder durch die Anordnung und/oder Form der Aussparungen asymmetrisch ist oder sind.

Durch die Brechung der Symmetrie des Kniespacers kann auf anatomisch begründete Asymmetrien der Gehbewegung und der dabei auftretenden Torsionskräfte Rücksicht genommen werden. Die Aussparungen können aber stattdessen auch symmetrisch zu der Ebene sein, die im eingesetzten Zustand parallel zur Sagittalebene des Patienten ist. Zur Aufnahme der nicht symmetrischen Torsionskräfte, die beim Gehen auf den eingesetzten Spacer wirken, ist es jedoch bevorzugt, dass die Aussparungen, über die die Torsionskräfte auf den ausgehärteten Knochenzement übertragen werden sollen, nicht symmetrisch zu der Ebene sind, die im eingesetzten Zustand parallel zur Sagittalebene des Patienten ist.

Des Weiteren kann vorgesehen sein, dass Aussparungen als Zylinder, Kegelstümpfe und/oder als mehrseitige Prismen ausgebildet sind, wobei zumindest eine Stirnseite der Zylinder, Kegelstümpfe und/oder Prismen zur Verankerungsfläche offen ist, insbesondere beide Stirnseiten der Zylinder, Kegelstümpfe und/oder Prismen zur Verankerungsfläche und zur Laufflächenseite offen sind, und wobei bevorzugt die Mantelflächen der Zylinder, der Kegelstümpfe und/oder Prismen in Längsrichtung geschlossen oder einfach in axialer Richtung durchbrochen sind.

Diese Symmetrien eignen sich gut zur Aufnahme der Kräfte bei Gehen und sind einfach zu fertigen. Die Symmetrie der Aussparungen kann erfindungsgemäß gegebenenfalls durch die Vorsprünge zur Verankerung des Knochenzements gebrochen sein.

Mit der Erfindung wird auch vorgeschlagen, dass mindestens ein erstes Führungselement auf der Laufflächenseite der Tibiakomponente angeordnet ist und mindestens ein zweites Führungselement auf der Laufflächenseite der Femurkomponente angeordnet ist, wobei die Führungselemente, im beim Patienten eingesetzten Zustand, durch Formschluss bei rotatorischer Bewegung der beiden Komponenten gegeneinander eine Verschiebung der Femurkomponente gegenüber der Tibiakomponente in Richtung eine Ebene, die parallel zur Sagittalebene ist, verhindern.

Durch die Führungselemente wird eine sichere Drehung der Komponenten gegeneinander nur um eine Achse gewährleistet, so dass eine unerwünschte Verdrehung des mit dem Kniespacer gebildeten Knies vermieden werden kann.

Zur Umsetzung erfindungsgemäßer Kniespacer kann vorgesehen sein, dass die Tibiakomponente und die Femurkomponente mit zumindest einem Kunststoff und/oder Metall aufgebaut sind, bevorzugt aus Kunststoff und/oder Metall bestehen, besonders bevorzugt aus Polymethylmethacrylat, ganz besonders bevorzugt aus mit Antibiotika dotierten ausgehärteten Polymethylmethacrylat-Knochenzement.

Diese Materialien eignen sich zur Herstellung erfindungsgemäßer Kniespacer besonders gut.

Ferner kann vorgesehen sein, dass die Tibiakomponente einen Schaft aufweist, der sich von einer zentralen Position der Verankerungsfläche mit einem Winkel zwischen 85° und 90° von der Verankerungsfläche aus erstreckt, bevorzugt senkrecht von der Verankerungsfläche aus erstreckt, und der Schaft zur Verankerung in einer Ausnehmung in der Tibia vorgesehen ist, wobei der Schaft bevorzugt von der Verankerungsfläche aus zumindest bereichsweise zusammenlaufend ist.

Der Schaft dient der Verankerung in der Tibia. Dadurch ist eine besonders stabile Verbindung zur Tibia erreichbar.

Es kann auch vorgesehen sein, dass auf der Verankerungsfläche wenigstens einer der Komponenten, bevorzugt beider Komponenten mehrere Abstandhalter angeordnet sind, wobei die Abstandhalter bevorzugt von der Verankerungsfläche aus zumindest bereichsweise zusammenlaufend sind, und besonders bevorzugt der Schaft zumindest vier Mal so hoch ist wie die Abstandhalter der Verankerungsfläche der Tibiakomponente.

Die Abstandhalter dienen dazu, dass eine ausreichende Menge und eine ausreichende Schichtdicke des Knochenzements zur Verbindung der Komponenten mit dem Knochen verbleiben und so eine stabile Verbindung der Komponenten mit den Knochen erzeugt wird.

Die Aufgaben der Erfindung werden auch gelöst durch ein Verfahren zur Herstellung eines Kniespacers, bei dem die Tibiakomponente und die Femurkomponente als separate Teile erzeugt werden und in der Tibiakomponente zumindest zwei Aussparungen ausgeformt werden und in der Femurkomponente zumindest zwei Aussparungen ausgeformt werden.

Die Aussparungen bewirken eine Stabilisierung des Aufbaus des Knies mit dem Kniespacer bei Gehbewegungen des Patienten.

Schließlich werden die Aufgaben der Erfindung auch gelöst durch die Verwendung eines solchen Kniespacers als temporärer Platzhalter in einem Knie eines Patienten.

Der Erfindung liegt die überraschende Erkenntnis zugrunde, dass es durch Aussparungen in den beiden Komponenten des Kniespacers gelingt, dass der Knochenzement in diesen Aussparungen aushärten kann und so eine mechanisch stabile Verbindung zwischen den Teilen des Kniespacers und dem verbindenden Knochenzement erzeugt, die dazu in der Lage ist, die beim Gehen auftretenden Torsionskräfte aufzunehmen, ohne dass sich der Knochenzement von den Komponenten des Kniespacers löst.

Die Erfindung liefert so einen zweiteiligen Kniespacer, bei dem während der Implantationszeit eine unerwünschte Torsion der Tibiakomponente und auch der Femurkomponente gegenüber dem zur temporären Verankerung eingesetzten Polymethylmethacrylat-Knochenzement weitgehend ausgeschlossen werden kann. Die Torsionskräfte werden durch die in den Aussparungen erzeugten Knochenzementausläufer gut und großflächig aufgenommen, so dass an keiner Stelle der Verbindung zwischen dem Kniespacer beziehungsweise den Komponenten des Kniespacers und dem Knochenzement Kraft- oder Drehmomentspitzen entstehen, die zur einer Zerstörung der Verbindung oder zu einem Brechen des Knochenzements führen würden.

Die Grundidee der Erfindung besteht darin, dass die Tibiakomponente und die Femurkomponente jeweils mindestens zwei Aussparungen besitzen, welche die distalen und die proximalen Oberflächen der jeweiligen Komponenten verbindet, so dass der zur Fixierung eingesetzte Polymethylmethacrylat-Knochenzement in die Aussparungen greifen kann, so dass es zu einem Formschluss zwischen dem Polymethylmethacrylat-Knochenzement und der Tibiakomponente und einem Formschluss zwischen dem Polymethylmethacrylat-Knochenzement und der Femurkomponente kommt. Dadurch, dass mindestens zwei Aussparungen in jeder Komponente angeordnet sind, ist eine Torsion der Tibiakomponente und der Femurkomponente gegenüber dem zur temporären Verankerung genutzten Polymethylmethacrylat-Knochenzement sicher ausgeschlossen. Wesentlich ist dabei auch, dass die Aussparungen sich nicht innerhalb der Laufflächen beziehungsweise Gleitflächen der Tibiakomponente und der Femurkomponente befinden. Wesentlich für die Erfindung ist auch, dass die Tibiakomponente derart zur Femurkomponente angeordnet ist, dass die Laufpaarung beziehungsweise Gleitpaarung der mindestens einen proximalen Lauffläche der Tibiakomponente mit der mindestens einen distalen Lauffläche der Femurkomponente nicht über Aussparungen der Tibiakomponente und der Femurkomponente verläuft.

Der erfindungsgemäße Kniespacer wird als temporärer Platzhalter im Rahmen von zweizeitigen septischen Revisionen von Kniegelenkendoprothesen verwendet.

Im Folgenden werden Ausführungsbeispiele der Erfindung anhand von fünf schematisch dargestellten Figuren erläutert, ohne jedoch dabei die Erfindung zu beschränken. Dabei zeigt:
Figur 1: eine schematische perspektivische Ansicht eines erfindungsgemäßen Kniespacers;
Figur 2: eine schematische perspektivische Ansicht der Tibiakomponente des erfindungsgemäßen Kniespacers;
Figur 3: eine schematische Seitenansicht der Femurkomponente des erfindungsgemäßen Kniespacers;
Figur 4: eine schematische Querschnittansicht des erfindungsgemäßen Kniespacers; und
Figur 5: eine schematische Querschnittansicht einer alternativen Tibiakomponente eines erfindungsgemäßen Kniespacers.

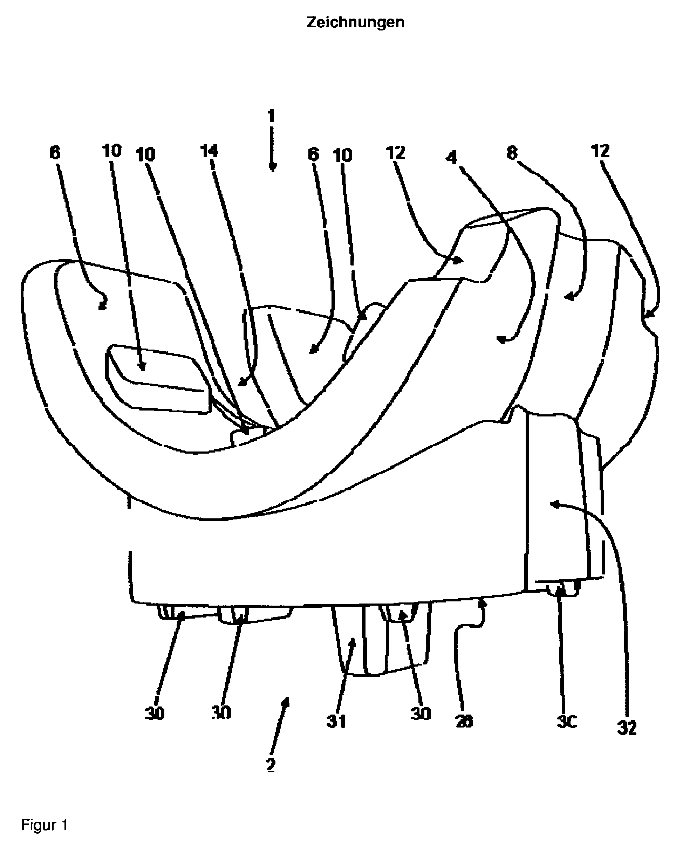

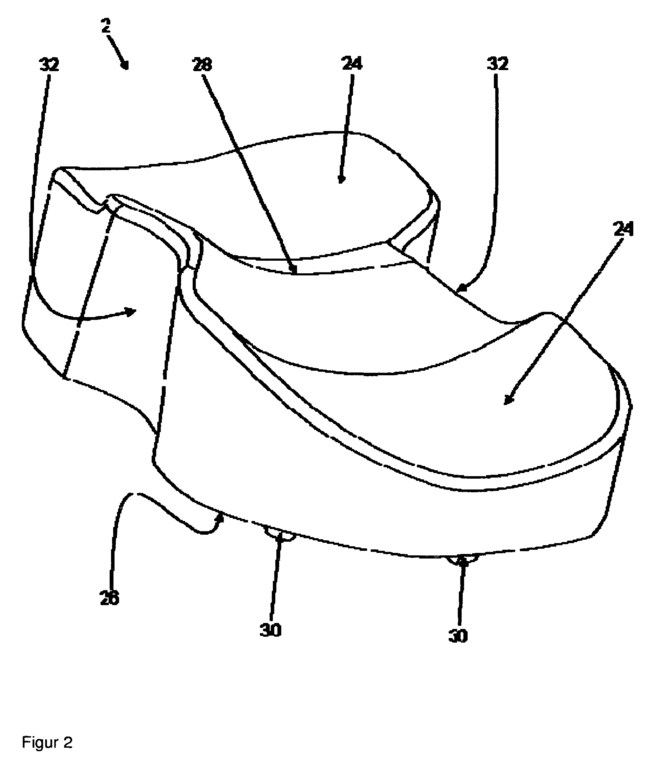

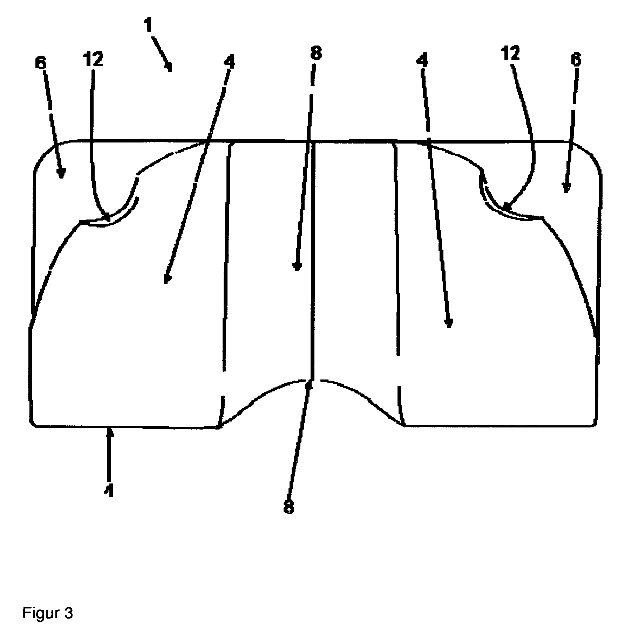

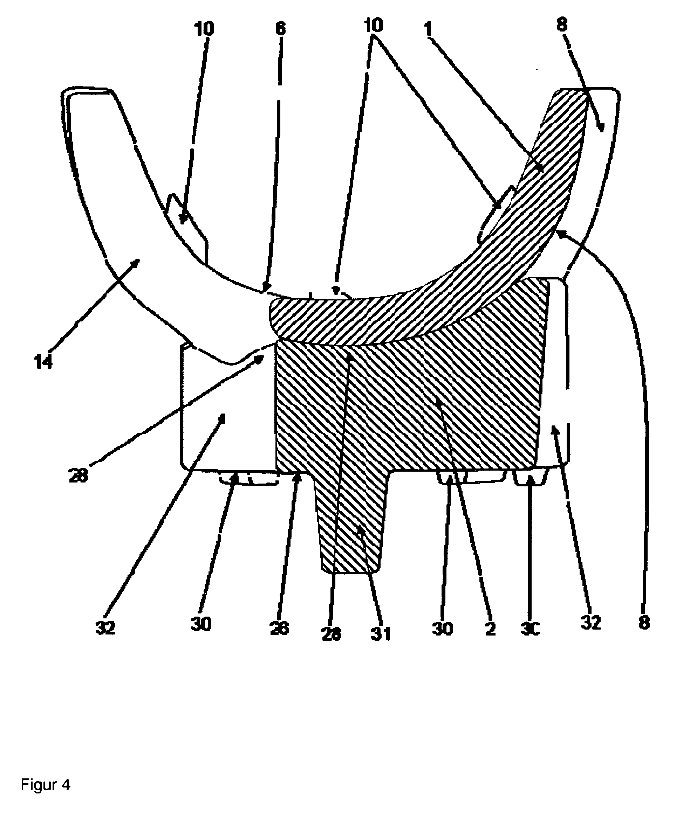

Figur 1 zeigt eine schematische perspektivische Ansicht eines erfindungsgemäßen Kniespacers. Der Kniespacer weist eine Femurkomponente 1 (in Figur 1 oben) und eine Tibiakomponente 2 (in Figur 1 unten) auf. Figur 2 zeigt eine schematische perspektivische Ansicht der Tibiakomponente 2 des erfindungsgemäßen Kniespacers nach Figur 1 und Figur 3 zeigt eine schematische Seitenansicht der Femurkomponente 1 des erfindungsgemäßen Kniespacers nach Figur 1 jeweils einzeln und separat. Figur 4 zeigt zudem eine schematische Querschnittansicht des erfindungsgemäßen Kniespacers nach Figur 1, wobei die geschnittenen Flächen schraffiert dargestellt sind.

Die Femurkomponente 1 weist eine nach Außen gewölbte Unterseite als Lauffläche 4 auf. Die der Lauffläche 4 gegenüberliegende Seite ist dementsprechend nach Innen gewölbt und bildet eine Verankerungsfläche 6 zur Befestigung der Femurkomponente 1 an einem Femur (Oberschenkelknochen - nicht dargestellt). Auf der Laufflächenseite (in den Figuren 1, 3 und 4 nach unten weisend) der Femurkomponente 1 ist zudem eine Vertiefung 8 als Führungselement vorgesehen. Die Lauffläche 4 ist zylindrisch geformt. Die Lauffläche 4 und die Vertiefung bilden ein gemeinsames Rotationskörpermantelsegment, so dass die Laufflächenseite auf einer passenden rotationssymmetrischen Innenwölbung drehbar ist und gleiten kann. Alternativ dazu aber weniger bevorzugt kann die Laufflächenseite auf einer ebenen oder weniger stark gewölbten Innenwölbung abrollen.

Auf der Verankerungsfläche 6 sind sechs Abstandhalter 10 vorgesehen, mit denen sichergestellt wird, dass eine Mindestmenge und eine Mindestdicke Knochenzement zwischen der Femurkomponente 1 und dem Femur vorhanden ist, wenn der Kniespacer bei einem Patienten eingesetzt ist. Die Abstandhalter 10 erstrecken sich parallel zueinander von der Verankerungsfläche 6 weg. Die Richtung, in der sich die Abstandhalter 10 von der Verankerungsfläche 6 weg erstrecken, entspricht der Lage des Femur, wenn die Femurkomponente 1 ordnungsgemäß beim Patienten eingesetzt ist. Die Abstandhalter 10 haben rechteckige Grundflächen mit abgerundeten Ecken. Die beiden mittleren Abstandhalter 10 bilden gerade allgemeine Zylinder mit abgerundet rechteckigen Grundflächen, während die vier anderen Abstandhalter 10 schiefe allgemeine Zylinder mit abgerundet rechteckigen Grundflächen sind.

An einem Ende der Femurkomponente 1 erstrecken sich zwei Aussparungen 12 von der Verankerungsfläche 6 bis zur gegenüberliegenden Laufflächenseite. Die Aussparungen 12 haben die Geometrie eines Zylindermantelsegments. Das Zylindermantelsegment der Aussparungen 12 umfasst etwa 45° eines vollständigen Zylindermartels. Die Zylindermantelsegmente der Aussparungen 12 weisen einen zueinander parallel angeordneten Bereich auf, zwischen dem ein äußerer Teil der Femurkomponente 1 angeordnet ist.

Wenn die Femurkomponente 1 an den Femur zementiert ist, erstrecken sich Ausläufer des gehärteten Knochenzements (nicht gezeigt) in die Aussparungen 12. Diese Ausläufer nehmen den äußeren Bereich der Femurkomponente 1 in die Zange und sorgen so für eine stabile Befestigung der Femurkomponente 1 am Femur, die dazu in der Lage ist, die beim Gehen auftretenden Kräfte, Torsionen und Drehmomente aufzunehmen, ohne dass es zu einer Beschädigung der Anbindung der Femurkomponente 1 an den Femur kommt.

Auf der den beiden Aussparungen 12 gegenüberliegenden Seite der Femurkomponente 1 ist eine breite schlitzförmige Aussparung 14 vorgesehen, die zum Einen wie auch die anderen Aussparungen 12 zur Verankerung genutzt wird, die aber auch gleichzeitig zur Aufnahme der Kreuzbänder des Patienten dient. Die Aussparung 14 und die Aussparungen 12 liegen einander derart gegenüber, dass eine stabile Befestigung durch die Ausläufer des Knochenzements bewirkt wird und so auch die zentralen Bereiche der Femurkomponente 1 in die Zange genommen werden.

Die Tibiakomponente 2 (unten) weist an der Oberseite (in den Figuren 1, 2 und 4 oben) eine Lauffläche 24 auf, die die gleiche Krümmung wie die Lauffläche 4 der Femurkomponente 1 aber als Innenwölbung hat. Die Laufflächen 4, 24 passen so formschlüssig ineinander.

Die Unterseite der Tibiakomponente 2 ist eben ausgestaltet und bildet die Verankerungsfläche 26 der Tibiakomponente 2, mit der die Tibiakomponente 2 an der Tibia (dem Schienbein - nicht gezeigt) befestigt werden kann. Als passendes Gegenstück zur Vertiefung 8 der Femurkomponente 1 weist die Oberseite der Tibiakomponente 2 eine Erhebung 28 als Führungselement auf. Die Lauffläche 24 und die Erhebung 28 bilden ein gemeinsames Rotationskörpermantelsegment 24, 28, das in das Rotationskörpermantelsegment 4, 8 der Femurkomponente 1 passt. Die Vertiefung 8 und die Erhebung 28 erstrecken sich dazu entlang des Umfangs der Rotationskörpermantelsegmente. Wenn die Femurkomponente 1 und die Tibiakomponente 2 beim Patienten eingesetzt sind und dadurch wie in den Figuren 1 und 4 gezeigt aneinander anliegen, können diese ineinander gleiten. Aufgrund der Führungselemente 8, 28 ist aber nur eine Drehung um eine Achse möglich, so dass durch den Kniespacer ein Kniegelenk nachgebildet wird.

An der Verankerungsfläche 26 der Tibiakomponente 2 sind acht nach unten senkrecht abstehende Abstandhalter 30 angeordnet, die ebenfalls dazu dienen, dass eine Mindestmenge und Mindestdicke Knochenzement zwischen der Tibia und der Tibiakomponente 2 angeordnet ist, wenn die Tibiakomponente 2 beim Patienten eingesetzt ist. Die Abstandhalter 30 sind leicht nach unten konisch zusammenlaufend geformt. In der Mitte der Verankerungsfläche 26 der Tibiakomponente 2 ist ein Schaft 31 vorgesehen, der sich ebenfalls senkrecht von der Verankerungsfläche 26 nach unten erstreckt und der ebenfalls leicht nach unten konisch zusammenläuft. Der Schaft 31 wird in eine entsprechende Kavität in der Tibia eingesetzt und dient der Stabilisierung des Kniespacers.

Sowohl der Schaft 31 als auch die Abstandhalter 30 sind Kegelstümpfe mit abgerundet rechteckigen Grundflächen und abgerundeten Kanten.

Auf zwei Seiten der Tibiakomponente 2 befinden sich randseitig zwei Aussparungen 32, die den gleichen Zweck erfüllen, wie die Aussparungen 12, 14 der Femurkomponente 1 (siehe oben). Die in Figur 2 nach rechts hinten weisende Aussparung 32 bildet ein Zylindermantelsegment mit etwa halbem Umfang (das heißt 180°) und dient wie de Aussparung 14 zusätzlich zur Aufnahme des Kreuzbands, wenn der Kniespacer beim Patienten eingesetzt ist. Die in Figur 2 nach vorne links ausgerichtete und auch in Figur 1 zu sehende Aussparung 32 läuft nach oben, in Richtung der Femurkomponente 1 konisch zusammen und bildet daher ein Kegelstumpfmantelflächensegment.

Die das Kegelstumpfmantelflächensegment bildende Aussparung 32 der Tibiakomponente 2 und zwei der Abstandhalter 30 sind nicht symmetrisch zu dem Mittelgrat der Erhebung 28, beziehungsweise zur einer der Sagittalebene parallelen Ebene des Kniespacers angeordnet, um die beim Gehen auftretenden Kräfte und Drehmomente passend zur Gehbewegung ableiten zu können. Die Erhebung 28 liegt also nur in etwa zwischen den Aussparungen 32 der Tibiakomponente 2.

Die Femurkomponente 1 und die Tibiakomponente 2 liegen als separate Teile vor und sind aus einem geeigneten Kunststoff gefertigt. Bevorzugt sind die beiden Komponenten 1, 2 aus einem mit Antibiotika dotierten ausgehärteten Polymethylmethacrylat-Knochenzement gefertigt. Mit dem gleichen Knochenzement werden die Komponenten 1, 2 auch beim Patienten eingesetzt und an der Tibia beziehungsweise dem Femur befestigt.

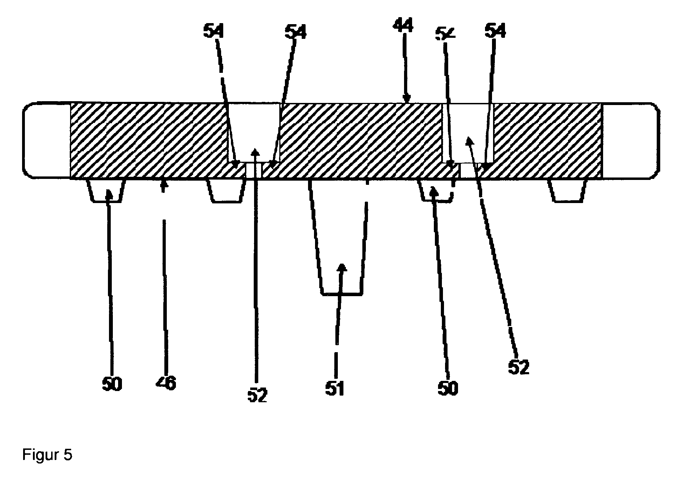

Figur 5 zeigt eine schematische Querschnittansicht einer alternativen Tibiakomponente eines erfindungsgemäßen Kniespacers. Die gezeigte Tibiakomponente hat eine ebene Lauffläche 44 als Oberseite (in Figur 5 oben) und eine dazu parallele ebene Verankerungsfläche 46 als Unterseite (in Figur 5 unten).

Auf der Verankerungsfläche 46 sind mehrere Abstandhalter 50 vorgesehen, die den gleichen Zweck wie die Abstandhalter 10, 30 nach den Figuren 1 bis 4 erfüllen sollen. Zudem ist auch an einer zentralen Position der Verankerungsfläche 46 der Tibiakomponente ein Schaft 51 vorgesehen, der dem gleichen Zweck dient, wie der Schaft 31 der Tibiakomponente 2 nach den Figuren 1 und 2.

Zudem weist die Tibiakomponente in dem geschnittenen dargestellten Bereich zwei Aussparungen 52 auf, die den gleichen Zweck erfüllen, wie die Aussparungen 32 der Tibiakomponente 2 nach den Figuren 1, 2 und 4. In den Aussparungen sind Vorsprünge 54 vorgesehen, die von der Verankerungsfläche 46 aus mit Knochenzement umflossen werden können und so zu einer noch stabileren Verankerung der Tibiakomponente an der Tibia führen. Das Prinzip kann erfindungsgemäß problemlos auch auf die Aussparungen einer Femurkomponente übertragen werden.

Anstatt scharfer Kanten und Ecken sind für Kniespacer abgerundete Ecken und Kanten bevorzugt.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Femurkomponente
- 2: Tibiakomponente
- 4: Lauffläche
- 6: Verankerungsfläche
- 8: Vertiefung
- 10: Abstandhalter
- 12: Aussparung
- 14: Aussparung
- 24: Lauffläche
- 26: Verankerungsfläche
- 28: Erhebung
- 30: Abstandhalter
- 31: Schaft
- 32: Aussparung
- 44: Lauffläche
- 46: Verankerungsfläche
- 50: Abstandhalter
- 51: Schaft
- 52: Aussparung
- 54: Vorsprung

## Patentansprüche

1. Kniespacer zum zeitweisen Ersetzen eines künstlichen Kniegelenks, wobei der Kniespacer eine Tibiakomponente (2) und eine Femurkomponente (1) als separate und, im beim Patienten eingesetzten Zustand, gegeneinander bewegliche Komponenten aufweist und die Tibiakomponente (2) und die Femurkomponente (1) jeweils mindestens eine Lauffläche (4, 24, 44) aufweisen, über die die Tibiakomponente (2) und die Femurkomponente (1) für den beim Patienten eingesetzten Zustand beweglich aneinander anlegbar sind, und die Tibiakomponente (2) eine Verankerungsfläche (26, 46) aufweist, die der Laufflächenseite gegenüber liegend angeordnet ist und die zur Verbindung der Tibiakomponente (2) mit der Tibia mittels eines Knochenzements vorgesehen ist und die Femurkomponente (1) eine Verankerungsfläche (6) aufweist, die der Laufflächenseite gegenüber liegend angeordnet ist und die zur Verbindung der Femurkomponente (1) mit dem Femur vorgesehen ist, **dadurch gekennzeichnet, dass** die Tibiakomponente (2) und die Femurkomponente (1) jeweils zumindest zwei Aussparungen (12, 14, 32, 52) aufweist, die sich von der Verankerungsfläche (6, 26, 46) in Richtung der Laufflächenseite bis in die Laufflächenseite erstrecken, wobei die Öffnungen der Aussparungen (12, 14, 32, 52) in der Laufflächenseite der Komponenten (1, 2) derart angeordnet sind, dass sie beim Artikulieren des Kniespacers nicht über die Lauffläche (4, 24, 44) der jeweils anderen Komponente (1, 2) gleiten.

2. Kniespacer nach Anspruch 1, **dadurch gekennzeichnet, dass**
die Aussparungen (12, 14, 32, 52) randseitig in der Tibiakomponente (2) und der Femurkomponente (1) angeordnet sind.

3. Kniespacer nach Anspruch 2, **dadurch gekennzeichnet, dass**
zumindest zwei der Aussparungen (12, 14, 32, 52) derart zueinander gegenüber liegend angeordnet sind, dass, im beim Patienten eingesetzten Zustand der Komponenten (1, 2), durch den Knochenzement in den Aussparungen (12, 14, 32, 52) geformte Ausläufer die Komponenten derart in die Zange nehmen, dass Bereiche der Komponenten (1, 2), bevorzugt zentrale Bereiche der Komponenten (1, 2) zwischen den Ausläufern angeordnet sind.

4. Kniespacer nach einem der vorangehenden Ansprüche, **dadurch gekennzeichnet, dass** sich die Aussparungen (12, 14, 32, 52) nicht in die Lauffläche (4, 24, 44) erstrecken.

5. Kniespacer nach einem der vorangehenden Ansprüche, **dadurch gekennzeichnet, dass** in den Aussparungen (12, 14, 32, 52) Vorsprünge (54) zur Verankerung des Knochenzements angeordnet sind.

6. Kniespacer nach einem der vorangehenden Ansprüche, **dadurch gekennzeichnet, dass** die Tibiakomponente (2) und/oder die Femurkomponente (1) asymmetrisch zu einer Ebene im eingesetzten Zustand ist oder sind, die parallel zur Sagittalebene des Patienten ist, vorzugsweise durch die Anordnung und/oder Form einer Aussparung (14, 32) zur Aufnahme des Kreuzbands des Patienten asymmetrisch ist oder sind und/oder durch die Anordnung und/oder Form der Aussparungen (12, 14, 32, 52) asymmetrisch ist oder sind.

7. Kniespacer nach einem der vorangehenden Ansprüche, **dadurch gekennzeichnet, dass** Aussparungen (12, 14, 32, 52) als Zylinder, Kegelstümpfe und/oder als mehrseitige Prismen ausgebildet sind, wobei zumindest eine Stirnseite der Zylinder, Kegelstümpfe und/oder Prismen zur Verankerungsfläche (6, 26, 46) offen ist, insbesondere beide Stirnseiten der Zylinder, Kegelstümpfe und/oder Prismen zur Verankerungsfläche (6, 26, 46) und zur Laufflächenseite offen sind, und wobei bevorzugt die Mantelflächen der Zylinder, der Kegelstümpfe und/oder Prismen in Längsrichtung geschlossen oder einfach in axialer Richtung durchbrochen sind.

8. Kniespacer nach einem der vorangehenden Ansprüche, **dadurch gekennzeichnet, dass** mindestens ein erstes Führungselement (28) auf der Laufflächenseite der Tibiakomponente (2) angeordnet ist und mindestens ein zweites Führungselement (8) auf der Laufflächenseite der Femurkomponente (1) angeordnet ist, wobei die Führungselemente (8, 28), im beim Patienten eingesetzten Zustand, durch Formschluss bei rotatorischer Bewegung der beiden Komponenten (1, 2) gegeneinander eine Verschiebung der Femurkomponente (1) gegenüber der Tibiakomponente (2) in Richtung eine Ebene, die parallel zur Sagittalebene ist, verhindern.

9. Kniespacer nach einem der vorangehenden Ansprüche, **dadurch gekennzeichnet, dass** die Tibiakomponente (2) und die Femurkomponente (1) mit zumindest einem Kunststoff und/oder Metall aufgebaut sind, bevorzugt aus Kunststoff und/oder Metall bestehen, besonders bevorzugt aus Polymethylmethacrylat, ganz besonders bevorzugt aus mit Antibiotika dotierten ausgehärteten Polymethylmethacrylat-Knochenzement.

10. Kniespacer nach einem der vorangehenden Ansprüche, **dadurch gekennzeichnet, dass** die Tibiakomponente (2) einen Schaft (31, 51) aufweist, der sich von einer zentralen Position der Verankerungsfläche (26, 46) mit einem Winkel zwischen 85° und 90° von der Verankerungsfläche (26, 46) aus erstreckt, bevorzugt senkrecht von der Verankerungsfläche (26, 46) aus erstreckt, und der Schaft (31, 51) zur Verankerung in einer Ausnehmung in der Tibia vorgesehen ist, wobei der Schaft (31, 51) bevorzugt von der Verankerungsfläche (26, 46) aus zumindest bereichsweise zusammenlaufend ist.

11. Kniespacer nach einem der vorangehenden Ansprüche, **dadurch gekennzeichnet, dass** auf der Verankerungsfläche (6, 26, 46) wenigstens einer der Komponenten (1,2), bevorzugt beider Komponenten (1, 2) mehrere Abstandhalter (10, 30, 50) angeordnet sind, wobei die Abstandhalter (10, 30, 50) bevorzugt von der Verankerungsfläche (6, 26, 46) aus zumindest bereichsweise zusammenlaufend sind, und besonders bevorzugt der Schaft (31, 51) zumindest vier Mal so hoch ist wie die Abstandhalter (30, 50) der Verankerungsfläche (26, 46) der Tibiakomponente (2).

12. Verfahren zur Herstellung eines Kniespacers nach einem der vorangehenden Ansprüche, **dadurch gekennzeichnet, dass** die Tibiakomponente (2) und die Femurkomponente (1) als separate Teile erzeugt werden und in der Tibiakomponente (2) zumindest zwei Aussparungen (12, 14, 32, 52) ausgeformt werden und in der Femurkomponente (1) zumindest zwei Aussparungen (12, 14, 32, 52) ausgeformt werden.

## Claims

1. A knee spacer for temporarily replacing an artificial knee joint, the knee spacer comprising a tibial component (2) and a femoral component (1) as separate components that can be moved relative to each other when introduced into the patient, and the tibial component (2) and the femoral component (1) each having at least one bearing surface (4, 24, 44), by way of which the tibial component (2) and the femoral component (1) can be placed against one another so as to be movable when introduced into the patient, and the tibial component (2) comprising an anchoring surface (26, 46), which is disposed opposite the bearing surface side and provided for connecting the tibial component (2) to the tibia by way of bone cement, and the femoral component (1) comprising an anchoring surface (6), which is disposed opposite the bearing surface side and provided for connecting the femoral component (1) to the femur, **characterized in that**
the tibial component (2) and the femoral component (1) each have at least two recesses (12, 14, 32, 52) extending from the anchoring surface (6, 26, 46) in the direction of the bearing surface side into the bearing surface side, the openings of the recesses (12, 14, 32, 52) being disposed in the bearing surface side of the components (1, 2) so as not to slide across the bearing surface (4, 24, 44) of the respective other component (1, 2) during articulation of the knee spacer.

2. The knee spacer according to claim 1, **characterized in that**
the recesses (12, 14, 32, 52) are provided on the periphery in the tibial component (2) and the femoral component (1).

3. The knee spacer according to claim 2, **characterized in that**
at least two of the recesses (12, 14, 32, 52) are disposed opposite from one another such that, when the components (1, 2) are introduced into the patient, extensions formed by the bone cement in the recesses (12, 14, 32, 52) sandwich the components such that regions of the components (1, 2), preferably central regions of the components (1, 2), are disposed between the extensions.

4. The knee spacer according to any one of the preceding claims, **characterized in that**
the recesses (12, 14, 32, 52) do not extend into the bearing surface (4, 24, 44).

5. The knee spacer according to any one of the preceding claims, **characterized in that** projections (54) for anchoring the bone cement are provided in the recesses (12, 14, 32, 52).

6. The knee spacer according to any one of the preceding claims, **characterized in that**
the tibial component (2) and/or the femoral component (1), when introduced, are asymmetrical to a plane that is parallel to the sagittal plane of the patient, preferably asymmetrical as a result of the arrangement and/or shape of a recess (14, 32) used to receive the cruciate ligament of the patient, and/or asymmetrical as a result of the arrangement and/or shape of the recesses (12, 14, 32, 52).

7. The knee spacer according to any one of the preceding claims, **characterized in that** recesses (12, 14, 32, 52) are designed as cylinders, truncated cones and/or multi-sided prisms, at least one end face of the cylinders, truncated cones and/or prisms being open toward the anchoring surface (6, 26, 46), in particular both end faces of the cylinders, truncated cones and/or prisms being open toward the anchoring surface (6, 26, 46) and toward the bearing surface side, and the lateral surfaces of the cylinders, the truncated cones and/or prisms preferably being closed in the longitudinal direction, or perforated once in the axial direction.

8. The knee spacer according to any one of the preceding claims, **characterized in that** at least one first guide element (28) is disposed on the bearing surface side of the tibial component (2), and at least one second guide element (8) is disposed on the bearing surface side of the femoral component (1), the guide elements (8, 28), when introduced into the patient, as a result of form fit preventing a displacement of the femoral component (1) with respect to the tibial component (2) in the direction of a plane parallel to the sagittal plane when the two components (1, 2) are moved in a rotatory manner.

9. The knee spacer according to any one of the preceding claims, **characterized in that**
the tibial component (2) and the femoral component (1) are made of at least one plastic material and/or metal, preferably consist of plastic material and/or metal, particularly preferably are made of polymethyl methacrylate, especially particularly preferably made of antibiotics-doped cured polymethyl methacrylate bone cement.

10. The knee spacer according to any one of the preceding claims, **characterized in that**
the tibial component (2) comprises a shaft (31, 51), which, from a central position of the anchoring surface (26, 46), extends at an angle between 85º and 90º from the anchoring surface (26, 46), preferably perpendicularly from the anchoring surface (26, 46), and the shaft (31, 51) is provided for anchoring in a recess in the tibia, the shaft (31, 51) preferably converging from the anchoring surface (26, 46) at least in regions.

11. The knee spacer according to any one of the preceding claims, **characterized in that**
a plurality of spacers (10, 30, 50) are disposed on the anchoring surface (6, 26, 46) of at least one of the components (1, 2), preferably of both components (1, 2), the spacers (10, 30, 50) preferably converging from the anchoring surface (6, 26, 46) at least in regions, and particularly preferably the shaft (31, 51) being at least four times as high as the spacers (30, 50) of the anchoring surface (26, 46) of the tibial component (2).

12. A method for producing a knee spacer according to any one of the preceding claims, **characterized in that**
the tibial component (2) and the femoral component (1) are produced as separate parts, and at least two recesses (12, 14, 32, 52) are formed in the tibial component (2), and at least two recesses (12, 14, 32, 52) are formed in the femoral component (1).

## Revendications

1. Espaceur pour genou pour le remplacement temporaire d'une prothèse de genou, où l'espaceur pour genou présente une partie de tibia (2) et une partie de fémur (1) séparées, servant de parties mobiles l'une par rapport à l'autre à l'état implanté chez le patient, et la partie de tibia (2) et la partie de fémur (1) présentent chacune au moins une surface de roulement (4, 24, 44), par l'intermédiaire de laquelle la partie de tibia (2) et la partie de fémur (1) peuvent être s'appuyer l'une sur l'autre en étant mobiles à l'état implanté chez le patient, et la partie de tibia (2) présente une surface d'ancrage (26, 46) qui est agencée en se situant en face de la face de la surface de roulement et est prévue pour la liaison de la partie de tibia (2) avec le tibia au moyen d'un ciment osseux, et la partie de fémur (1) présente une surface d'ancrage (6) qui est agencée se situant en face de la face de la surface de roulement et qui est prévue pour la liaison de la partie de fémur (1) avec le fémur, **caractérisé en ce que**
la partie de tibia (2) et la partie de fémur (1) présentent chacune au moins deux évidements (12, 14, 32, 52) qui s'étendent à partir de la surface d'ancrage (6, 26, 46) en direction de la face de la surface de roulement jusque dans la face de la surface de roulement, où les ouvertures des évidements (12, 14, 32, 52) sont disposées dans la face de la surface de roulement des parties (1, 2) de telle manière qu'elles ne glissent pas par-dessus la surface de roulement (4, 24, 44) de l'autre partie (1, 2) correspondante lors du fonctionnement de l'articulation de l'espaceur pour genou.

2. Espaceur pour genou selon la revendication 1, **caractérisé en ce que** les évidements (12, 14, 32, 52) sont agencés sur le bord dans la partie de tibia (2) et la partie de fémur (1).

3. Espaceur pour genou selon la revendication 2, **caractérisé en ce qu'**au moins deux des évidements (12, 14, 32, 52) sont agencés l'un par rapport à l'autre de telle manière que, dans l'état implanté des parties (1, 2) chez le patient, des ramifications formées par le ciment osseux dans les évidements (12, 14, 32, 52) enserrent comme une pince les parties, de sorte que des régions des parties (1, 2), de préférence des régions centrales des parties (1, 2), sont disposées entre les ramifications.

4. Espaceur pour genou selon l'une des revendications précédentes, **caractérisé en ce que**
les évidements (12, 14, 32, 52) ne s'étendent pas dans la surface de roulement (4, 24, 44).

5. Espaceur pour genou selon l'une des revendications précédentes, **caractérisé en ce que**
des parties saillantes (54) sont agencées dans les évidements (12, 14, 32, 52) pour l'ancrage du ciment osseux.

6. Espaceur pour genou selon l'une des revendications précédentes, **caractérisé en ce que**
la partie de tibia (2) et/ou la partie de fémur (1) est ou sont disposée(s) de manière asymétrique par rapport à un plan à l'état implanté, plan qui est parallèle au plan sagittal du patient, de préférence, est ou sont disposée(s) de manière asymétrique par l'agencement et/ou la forme d'un évidement (14, 32) pour la réception du ligament croisé du patient, et/ou est ou sont disposée(s) de manière asymétrique par l'agencement et/ou la forme des évidements (12, 14, 32, 52).

7. Espaceur pour genou selon l'une des revendications précédentes, **caractérisé en ce que**
des évidements (12, 14, 32, 52) sont conçus sous la forme de cylindres, de cônes tronqués et/ou de prismes à plusieurs faces, où au moins une face frontale des cylindres, des cônes tronqués et/ou des prismes est ouverte vers la surface d'ancrage (6, 26, 46), en particulier, deux faces frontales des cylindres, des cônes tronqués et/ou des prismes sont ouvertes vers la surface d'ancrage (6, 26, 46) et vers la face de la surface de roulement, et où, de préférence, les surfaces d'enveloppe des cylindres, des cônes tronqués et/ou des prismes sont fermées dans la direction longitudinale ou simplement percées dans la direction axiale.

8. Espaceur pour genou selon l'une des revendications précédentes, **caractérisé en ce qu'**au moins un premier élément de guidage (28) est agencé sur la face de la surface de roulement de la partie de tibia (2) et au moins un deuxième élément de guidage (8) est agencé sur la face de la surface de roulement de la partie de fémur (1), où les éléments de guidage (8, 28), à l'état implanté chez le patient, empêchent un déplacement de la partie de fémur (1) par rapport à la partie de tibia (2) en direction d'un plan qui est parallèle au plan sagittal par un crabotage des deux parties (1, 2) l'une contre l'autre lors d'un mouvement de rotation.

9. Espaceur pour genou selon l'une des revendications précédentes, **caractérisé en ce que**
la partie de tibia (2) et la partie de fémur (1) sont construites avec au moins une matière plastique et/ou du métal, de préférence sont constituées en matière plastique et/ou en métal, de manière particulièrement préférée en polyméthacrylate de méthyle, de manière tout particulièrement préférée en un ciment osseux de polyméthacrylate de méthyle durci dopé avec des antibiotiques.

10. Espaceur pour genou selon l'une des revendications précédentes, **caractérisé en ce que**
la partie de tibia (2) présente une tige (31, 51) qui s'étend à partir d'une position centrale de la surface d'ancrage (26, 46) avec un angle entre 85° et 90 ° à partir de la surface d'ancrage (26, 46), de préférence s'étend verticalement à partir de la surface d'ancrage (26, 46), et la tige (31, 51) est prévue pour l'ancrage dans un évidement dans le tibia, où la tige (31, 51) est de préférence au moins partiellement en pointe à partir de la surface d'ancrage (26, 46).

11. Espaceur pour genou selon l'une des revendications précédentes, **caractérisé en ce que**
plusieurs entretoises (10, 30, 50) sont disposées sur la surface d'ancrage (6, 26, 46) d'au moins une des parties (1, 2), de préférence des deux parties (1, 2), où les entretoises (10, 30, 50) sont de préférence au moins partiellement en pointe à partir de la surface d'ancrage (6, 26, 46), et de manière particulièrement préférée, la tige (31, 51) est quatre fois aussi haute que les entretoises (30, 50) de la surface d'ancrage (26, 46) de la partie de tibia (2).

12. Procédé de fabrication d'un espaceur pour genou selon l'une des revendications précédentes, **caractérisé en ce que**
la partie de tibia (2) et la partie de fémur (1) sont faites sous forme de parties séparées et, dans la partie de tibia (2), au moins deux évidements (12, 14, 32, 52) sont formés, et au moins deux évidements (12, 14, 32, 52) sont formés dans la partie de fémur (1).
